Europäisches Patentamt

European Patent Office

Office européen des brevets

(11) Veröffentlichungsnummer: **0 288 857**
**A2**

(12) **EUROPÄISCHE PATENTANMELDUNG**

(21) Anmeldenummer: **88106134.5**

(22) Anmeldetag: **18.04.88**

(51) Int. Cl.⁴: **C07C 85/04**

(30) Priorität: **27.04.87 DE 3713996**

(43) Veröffentlichungstag der Anmeldung:
**02.11.88 Patentblatt 88/44**

(84) Benannte Vertragsstaaten:
**AT BE CH DE ES FR GB GR IT LI NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Rutzen, Horst, Dr.**
**Falkenweg 12**
**D-4018 Langenfeld(DE)**
Erfinder: **Lorenz, Peter, Dr.**
**Poststrasse 56**
**D-4018 Langenfeld(DE)**
Erfinder: **Tesmann, Holger, Dr.**
**Vennstrasse 61**
**D-4000 Düsseldorf 12(DE)**

(54) **Verfahren zur Herstellung von quartären Ammoniumhalogeniden.**

(57) Quartäre Ammoniumhalogenide in Pulver-oder Granulatform mit definierter Teilchenstruktur lassen sich unter geringem apparativen Aufwand erhalten, indem man tertiäre Amine mit Alkylhalogeniden im Molverhältnis von 1 : 3 bis 1 : 8 bei Temperaturen von 50 bis 150°C unter Überdruck in Abwesenheit von Lösemitteln in einem mit einem Rührwerk versehenen und gegebenenfalls ein Bodenventil oder Tauchrohr aufweisenden Reaktor, wobei das Rührwerk im wesentlichen die gesamte Reaktoroberfläche eng überstreicht, umsetzt, wobei die Alkylhalogenide auf Reaktionstemperatur erwärmt in dem Reaktor vorgelegt werden, die tertiären Amine unter Rühren mit einer solchen Geschwindigkeit eingespeist werden, daß der Sollwert der Reaktionstemperatur um nicht mehr als 20°C unter-bzw. überschritten wird und man nach Beendigung der Reaktion die Alkylhalogenide bei einer Autoklaventemperatur zwischen 50°C und der gewählten Reaktionstemperatur entspannt.

## Verfahren zur Herstellung von quartären Ammoniumhalogeniden

Die Erfindung betrifft ein Verfahren zur Herstellung von quartären Ammoniumhalogeniden in Pulver-oder Granulatform durch Umsetzung von tertiären Aminen mit Alkylhalogeniden im Molverhältnis von 1 : 3 bis 1 : 8 bei Temperaturen von 50 bis 150°C unter Überdruck in Abwesenheit von Lösemitteln in einem mit einem Rührwerk versehenen Reaktor, wobei das Rührwerk im wesentlichen die gesamte Reaktoroberfläche eng überstreicht.

Quartäre Ammoniumhalogenide werden üblicherweise durch Umsetzung von tertiären Aminen mit Alkylhalogeniden hergestellt. Diese Reaktion wird normalerweise in polaren Lösemitteln wie Wasser, Ethanol, Isopropanol oder Gemischen derselben durchgeführt. Besonders bei der Herstellung von quartären Ammoniumhalogeniden mit einem längerkettigen Alkylrest werden wegen der begrenzten Löslichkeit dieser Verbindungen und ihrer Tendenz, bei höheren Konzentrationen eine Gelbildung zu verursachen, beträchtliche Mengen an Lösemitteln benötigt.

Führt man die Quaternierung in Abwesenheit eines Lösemittels und in Gegenwart eines Überschusses des Alkylierungsmittels durch, kommt es beim Einsatz üblicher Reaktoren zu der Ausbildung eines festen Kuchens der quartären Ammoniumsalze, der nur mühsam aus dem Reaktor entfernt werden kann. Zur Lösung dieses technischen Problems ist bereits in der DE-OS 28 53 241 ein Verfahren beschrieben worden, gemäß dem das Alkylierungsprodukt zusammen mit dem überschüssigen Alkylierungsmittel dem Autoklaven entnommen und mit Hilfe eines Tangentialabscheiders in Pulver-oder Granulatform isoliert wird. Dieses Verfahren ist insofern mit einem erheblichen apparativen Aufwand verbunden, als der hier eingesetzte Tangentialabscheider mit einer komplizierten Druckregeleinrichtung versehen sein muß, um eine sichere Rückführung des überschüssigen Alkylhalogenids zu gewährleisten.

Es wurde nun gefunden, daß man in einer wesentlich einfacheren Apparatur ohne Tangentialabscheider ebenfalls zu quartären Ammoniumhalogeniden in Pulver-oder Granulatform kommt, wenn man spezielle Verfahrensparameter, insbesondere die Reaktionstemperatur und Abweichungen von derselben und die Temperatur, bei der das Reaktionssystem nach beendigter Reaktion entspannt wird, einhält.

Demgemäß ist die Erfindung auf ein Verfahren der eingangs genannten Art gerichtet, gemäß dem man

a) in dem Reaktor die auf Reaktionstemperatur erwärmten Alkylhalogenide vorlegt,

b) die tertiären Amine unter Rühren mit einer solchen Geschwindigkeit einspeist, daß der Sollwert der Reaktionstemperatur um nicht mehr als 20°C unter-bzw. überschritten wird und

c) nach Beendigung die Alkylhalogenide bei einer Reaktortemperatur zwischen 50°C und der jeweils gewählten Reaktionstemperatur entspannt und die pulverförmigen quartären Ammoniumhalogenide dem Reaktor entnimmt.

Das vorstehend erläuterte Verfahren der Erfindung weist den zusätzlichen Vorteil auf, daß man über die Reaktionstemperatur die Kornstruktur des erhaltenen Ammoniumhalogenids vorgeben kann; so werden bei tieferen Reaktionstemperaturen pulverförmige Produkte, bei höherer Temperatur solche in Granulatform, z.B. mit einer Reiskornstruktur, erhalten; dies bietet anwendungstechnische Vorteile für die quartären Ammoniumhalogenide, z.B. bei ihrer Dosierung.

Das Verfahren der Erfindung arbeitet bei Reaktionstemperaturen, die auf beliebige Bereiche zwischen 50 und 150°C, vorzugsweise 70 bis 120°C, einstellbar sind. In einer bevorzugten Ausführungsform der Erfindung werden Reaktoren eingesetzt, in die die Alkylhalogenide über ein Bodenventil am Reaktorboden bzw. über ein Tauchrohr in der Nähe des Reaktorbodens wenig oberhalb des Rührwerkes eingespeist werden.

Vorzugsweise werden bei der Durchführung des Verfahrens der Erfindung als Alkylhalogenide insbesondere Methylchlorid, Methylbromid, Methyljodid, Ethylchlorid und Ethylbromid eingesetzt.

Das Verfahren der Erfindung eignet sich insbesondere für die Quarternierung von tertiären Aminen, die außer einem 2-Alkyl-oder Hydroxyalkylrest mit 1 bis 3 Kohlenstoffatomen zwei oder einen Alkylrest oder Hydroxyalkylrest mit 8 bis 18 Kohlenstoffatomen enthalten.

Als Beispiel für geeignete tertiäre Amine mit einem längerkettigen Alkylrest können die folgenden genannt werden: Dimethyl-octylamin, Dimethyl-decylamin, Diethyl-decylamin, Dimethyl-dodecylamin, Di-n-propyl-dodecylamin, Diethyl-tetradecylamin, Dimethyl-hexadecylamin, Di-n-propyl-hexadecylamin, Dimethyl -octadecylamin, Bis-(2-hydroxyethyl)-decylamin, Bis-(2-hydroxypropyl)-dodecyl-amin, Bis-(2-hydroxyethyl)-hexadecylamin, Bis-(2-hydroxyethyl)-octadecylamin und dergleichen.

Als Ausgangsmaterial für das Verfahren der Erfindung eignen sich weiterhin auch tertiäre Amine oder Gemische von tertiären Aminen mit einem längerkettigen Hydroxyalkylrest, wie sie nach üblichen Verfahren aus längerkettigen Epoxyalkanen oder Epoxyalkangemischen mit 8 bis 18 Kohlenstoffatomen und terminalen oder nicht-terminalen Epoxygruppen durch Umsetzung mit Dialkylaminen wie Dimethylamin, Diethylamin und Di-n-propylamin sowie durch Umsetzung von Dihydroxyalkylaminen wie Diethanolamin und Dipropanolamin erhältlich sind. Bei der Herstellung dieser tertiären Amine werden vor allem Gemische von Epoxyalkanen mit unterschiedlicher Kettenlänge als Ausgangsstoffe eingesetzt. Bei den Epoxyalkanen mit nicht-terminaler Epoxygruppierung werden überdies Gemische solcher Verbindungen bevorzugt, in denen Epoxygruppen willkürlich über die an der Kohlenwasserstoffkette vorhandenen Innenpositionen verteilt sind. Beispiele für derartige tertiäre Amine sind Dimethyl-2-hydroxydecylamin, Bis-(2-hydroxy-ethyl)-2-hydroxydecylamin, Diethyl-2-hydroxy-dodecylamin und Bis-(2-hydroxyethyl)-2-hydroxy-dodecylamin; weiterhin Umsetzungsprodukte von Dimethylamin mit einem 1,2-Epoxyalkangemisch der Kettenlänge $C_{12}$ bis $C_{14}$, das Umsetzungsprodukt von Diethylamin mit einem 1,2-Epoxyalkangemisch der Kettenlänge $C_{14}$ bis $C_{16}$, das Umsetzungsprodukt von Di-n-propylamin mit einem 1,2-Epoxyalkangemisch der Kettenlänge $C_{16}$ bis $C_{18}$, das Umsetzungsprodukt von Diethanolamin mit einem 1,2-Epoxyalkangemisch der Kettenlänge $C_{14}$ bis $C_{16}$, das Umsetzungsprodukt von Dimethylamin mit einem Epoxyalkangemisch der Kettenlänge $C_{11}$ bis $C_{14}$ mit statistisch verteilten, nicht-terminalen Epoxygruppierungen, das Umsetzungsprodukt von Diethylamin mit einem Epoxyalkangemisch der Kettenlänge $C_{14}$ bis $C_{16}$ mit statistisch verteilten, nicht-ter minalen Epoxygruppierungen, das Umsetzungsprodukt von Di-n-propylamin mit einem Epoxyalkangemisch der Kettenlänge $C_{15}$ bis $C_{18}$ mit statistisch verteilten, nicht-terminalen Epoxyalkangruppierungen sowie das Umsetzungsprodukt von Diethanolamin mit einem Epoxyalkangemisch der Kettenlänge $C_{11}$ bis $C_{14}$ mit statistisch verteilten, nicht-terminalen Epoxygruppierungen.

Gemäß einem weiteren vorteilhaften Merkmal der Erfindung entspannt man nach Beendigung der Reaktion das überschüssige Alkylhalogenid in eine Rückgewinnungsanlage.

Das Verfahren der Erfindung wird in üblichen Reaktoren, beispielsweise in normalen Rührautoklaven oder in auf Überdruck (30 bar) ausgelegten speziellen Knetern (Druvatherm®) durchgeführt, die mit einer Heiz-und Kühleinrichtung, einem Rührwerk, einer geeigneten Zuführung für das tertiäre Amin, beispielsweise einem Tauchrohr sowie einem geeigneten Ventil zum Ablassen des überschüssigen Alkylhalogenids nach Beendung der Reaktion ausgestattet sind. In einer speziellen Ausführungsform der Erfindung ist der Reaktor mit einem Bodenventil zur Einspeisung des tertiären Amins ausgestattet.

Das Verfahren der Erfindung weist somit die folgenden wesentlichen Merkmale auf:

1. Zunächst wird das Alkylhalogenid, z.B. Methylchlorid, vorgelegt und auf eine vorbestimmte Reaktionstemperatur im Bereich von 50 bis 150, vorzugsweise 70 bis 130°C erhitzt. Dabei wird ein Überschuß des Alkylhalogenids verwendet, damit nach Beendigung der Reaktion eine rührfähige Paste erhalten wird.

2. Die Gesamtmenge des umzusetzenden Tertiäramins wird nicht auf einmal in den Reaktor gegeben, sonder mit einer Geschwindigkeit zudosiert, daß die Reaktionswärme (ca. 35 kcal/mol) abgeführt werden kann, wobei der Sollwert der Reaktionstemperatur um nicht mehr als 20°C unter-bzw. überschritten wird.

3. Der Rührer ist in der Weise ausgebildet, daß er weitgehend die gesamte Oberfläche, insbesondere die Boden-und Seitenflächen, überstreicht und möglichst nahe an der Wandung entlangläuft.

4. Das Alkylhalogenid wird nach beendeter Reaktion bei einer Autoklaventemperatur zwischen 50°C und der gewählten Reaktionstemperatur, vorzugsweise bei der Reaktionstemperatur, entspannt.

5. Die Zufuhr des Tertiäramins erfolgt bevorzugt über ein Bodenventil oder ein Tauchrohr in der Nähe des Bodens.

Die Erfindung wird im folgenden anhand von bevorzugten Ausführungsbeispielen für die Herstellung von Trimethyl-cetylammoniumchlorid näher erläutert.

Beispiel 1

Ein 25 1-Autoklav mit Drehrührer und Stopfbüchse wurde evakuiert und mit 3,45 kg (68,3 Mol) Methylchlorid beschickt. Anschließend wurde der Autoklav auf 80°C erwärmt, wobei sich ein Druck von 18 bar einstellte.

Anschließend wurden mittels einer Kolbenpumpe durch ein Tauchrohr innerhalb von 45 min 5,00 kg (19,86 Mol) Dimethylcetylamin (Aminzahl 222,8, berechnetes Molekulargewicht 251,8) gegeben. Während des Nachrührens (45 min) stieg die Autoklaventemperatur auf 92°C. Anschließend wurde das überschüssige Methylchlorid unter fortgesetztem Rühren in eine Rückgewinnungsanlage entspannt. Nach 30 min war der Druck auf 1 bar und gleichzeitig die Temperatur auf 84°C gefallen.

Das Endprodukt wurde als farbloses Pulver mit einem Schüttgewicht von 614 g/l, einer Aminzahl von 0,4 und einer Säurezahl von 1,4 erhalten. Eine 25 %-ige wäßrige Lösung war klar. Die Ausbeute war quantitativ (6 kg).

Beispiel 2.

Es wurde die gleiche Reaktion wie gemäß Beispiel 1, jedoch bei höherer Temperatur, durchgeführt. Das Methylchlorid wurde auf 100°C erhitzt. Beim Einspeisen des Dimethylcetylamins stieg die Temperatur auf 115°C und der Methylchloriddruck auf 27,5 bar. Nach 30 min Nachrühren wurde unter fortgesetztem Rühren entspannt. Innerhalb von 30 min sank der Druck auf 1 bar und die Temperatur auf 111°C.

Die Ausbeute war quantitativ. Das Produkt wies eine Aminzahl von 1,0 und eine Säurezahl von 1,2 bei einem Schüttgewicht von 593 g/l auf. Das Endprodukt wurde in Form einer Reiskornstruktur erhalten.

## Ansprüche

1. Verfahren zur Herstellung von quartären Ammoniumhalogeniden in Pulver-oder Granulatform durch Umsetzung von tertiären Aminen mit Alkylhalogeniden im Molverhältnis von 1 : 3 bis 1 : 8 bei Temperaturen von 50 bis 150°C unter Überdruck in Abwesenheit von Lösemitteln in einem mit einem Rührwerk versehenen Reaktor, wobei das Rührwerk im wesentlichen die gesamte Reaktoroberfläche eng überstreicht, dadurch gekennzeichnet, daß man

a) in dem Reaktor die auf Reaktionstemperatur erwärmten Alkylhalogenide vorlegt,

b) die tertiären Amine unter Rühren mit einer solchen Geschwindigkeit einspeist, daß der Sollwert der Reaktionstemperatur um nicht mehr als 20°C unter-bzw. überschritten wird und

c) nach Beendigung der Reaktion die Alkylhalogenide bei einer Reaktiortemperatur zwischen 50°C und der gewählten Reaktionstemperatur entspannt und die pulverförmigen quartären Ammoniumhalogenide dem Reaktor entnimmt.

2. Verfahren nach Anspruch 1, dadurch gekennzeichnet, daß man die tertiären Amine über ein Bodenventil am Reaktorboden oder über ein Tauchrohr in der Nähe des Reaktorbodens einspeist.

3. Verfahren nach Anspruch 1 oder 2, gekennzeichnet durch eine Reaktionstemperatur von 70 bis 120°C.

4. Verfahren nach einem der Ansprüche 1 bis 3, dadurch gekennzeichnet, daß man als Alkylhalogenide Methylchlorid, Methylbromid, Methyljodid, Ethylchlorid oder Ethylbromid verwendet.

5. Verfahren nach einem der Ansprüche 1 bis 4, dadurch gekennzeichnet, daß man tertiäre Verbindungen verwendet, die außer einem oder zwei 2-Alkyl-oder Hydroxyalkylresten mit 1 bis 3 Kohlenstoffatomen zwei oder einen Alkylrest oder Hydroxyalkylrest mit 8 bis 18 Kohlenstoffatomen enthalten.

6. Verfahren nach einem der Ansprüche 1 bis 5, dadurch gekennzeichnet, daß man das überschüssige Alkylhalogenid in eine Rückgewinnungsanlage entspannt.